(19) Europäisches Patentamt / European Patent Office / Office européen des brevets

(11) **EP 4 106 771 B1**

(12) **EUROPEAN PATENT SPECIFICATION**

(45) Date of publication and mention
of the grant of the patent:
**12.02.2025 Bulletin 2025/07**

(21) Application number: **21711957.7**

(22) Date of filing: **17.02.2021**

(51) International Patent Classification (IPC):
*A61K 36/9068* *(2006.01)*    *A61K 36/45* *(2006.01)*
*A61K 36/185* *(2006.01)*    *A61K 31/728* *(2006.01)*
*A61P 1/04* *(2006.01)*

(52) Cooperative Patent Classification (CPC):
(C-Sets available)
**A61P 1/04; A61K 31/728; A61K 36/185;
A61K 36/9068**                                      (Cont.)

(86) International application number:
**PCT/IB2021/051329**

(87) International publication number:
**WO 2021/165846 (26.08.2021 Gazette 2021/34)**

(54) **COMPOSITION FOR THE PROTECTION OF THE GASTROINTESTINAL MUCOSA AND FOR THE PREVENTION AND TREATMENT OF DISEASES ASSOCIATED THEREWITH**

ZUSAMMENSETZUNG ZUM SCHUTZ DER GASTOINTESTINALEN SCHLEIMHAUT AND ZUR VORBEUGUNG UND BEHANDLUNG VON DAMIT VERBUNDENEN ERKRANKUNGEN

COMPOSITION POUR LA PROTECTION DE LA MUQUEUSE GASTRO-INTESTINALE ET POUR LA PRÉVENTION ET LE TRAITEMENT DE MALADIES ASSOCIÉES À CELLE-CI

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.02.2020 IT 202000003092**

(43) Date of publication of application:
**28.12.2022 Bulletin 2022/52**

(73) Proprietor: **Neilos S.r.l.
80063 Piano di Sorrento (NA) (IT)**

(72) Inventor: **DI MAIO, Umberto
80065 Sant'Agnello (Napoli) (IT)**

(74) Representative: **Di Giovine, Paolo et al
Società Italiana Brevetti S.p.A.
Piazza di Pietra, 38-39
00186 Roma (IT)**

(56) References cited:
**EP-A1- 2 545 925          WO-A1-2010/083967
WO-A1-2010/083968     WO-A1-2017/158041**

• **DATABASE WPI Week 201768, Derwent World
Patents Index; AN 2017-65826V, XP002800428**

Remarks:
The file contains technical information submitted
after the application was filed and not included in this
specification

(52) Cooperative Patent Classification (CPC): (Cont.)

C-Sets
**A61K 31/728, A61K 2300/00;**
**A61K 36/185, A61K 2300/00;**
**A61K 36/9068, A61K 2300/00**

## Description

The present invention relates to a composition of substances preferably obtained from natural sources, which is effective in the protection of the gastrointestinal mucosa and in the prevention and treatment of diseases associated therewith. The invention is defined by the appended claims.

Nowadays, gastric disorders are more and more common and debilitating. A significant portion of the world's population (3-28%), including mainly women and the elderly, is subject to gastric disorders and sometimes so severely as to affect the individual's quality of life. Gastric disorders are intended to mean gastritis, gastroesophageal reflux, gastric hyperacidity, delay in the process of gastric emptying, heartburn and dyspepsia. Studies carried out especially over the last decade have highlighted some possible risk factors for the development of typical digestive disorders: among these, the first appears to be *Helicobacter pylori,* and the second place appears to be occupied by smoking, alcohol abuse, stress, obesity and pregnancy. However, eating habits, the way of eating and what is eaten certainly also have an important effect on the gastric contents and the motility of the digestive tract. Some drugs, especially if taken for prolonged periods of time, can alter the gastric secretion mechanisms, resulting in increased hydrochloric acid concentration in the stomach, and consequently cause gastric hyperacidity. Dyspepsia, generally defined as "bad digestion", indicates a pathological condition characterised by the predominant presence of persistent or recurrent pain and/or discomfort localized in the epigastrium. This gastric disorder can be classified into: (i) secondary dyspepsia mainly caused by diseases of the upper digestive tract such as esophagitis, gastritis, or by prolonged periods of substance or drug abuse; (ii) primary or functional dyspepsia which, on the other hand, is not attributable to any particular cause and is a chronic and/or recurrent disorder characterised by pain and discomfort in the upper abdomen. Many subjects suffering from dyspepsia experience an increase in gastric emptying time, mainly due to altered gastric motility. The onset of this condition results in prolonged permanence of the ingested food, therefore the digestive process is slowed down. More generally, typical symptoms of dyspepsia include premature satiety during meals, feeling of fullness, abdominal bloating and discomfort, heartburn, and gastric hyperacidity. The term "hyperacidity" refers to a condition characterised by an increase in the acidity of the gastric contents of the stomach compared to the physiological values ranging from 1.5 to 2, and by a set of clinical symptoms such as a sense of fullness, distress and burning of the epigastrium, often associated with regurgitation of acidic material. One of the causes of gastric hyperacidity is abnormal hormonal secretion during and after meals, for example hypergastrinemia. Gastrin is a hormone which, by acting on the CCK2 receptors of the parietal cells of the stomach, stimulates the production of histamine which, in turn, activates acid secretion. Gastric hyperacidity is a condition predisposing to a whole series of alterations of the normal gastroesophageal physiology. Hyperacidity, in fact, increases the risk of peptic ulcers and gastroesophageal reflux. Gastroesophageal reflux syndrome, commonly referred to as GERD, is an ever-increasing clinical problem. This syndrome is defined as a set of uncomfortable symptoms and complications due to the reflux of gastric contents into the esophagus. Although one of the main etiological factors is gastric hyperacidity, relaxation of the lower gastroesophageal sphincter is a factor frequently encountered in the course of GERD. The classic symptom of gastroesophageal reflux is heartburn, which manifests itself as a burning and heat sensation in the chest that radiates towards the mouth. Gastric hyperacidity and gastroesophageal reflux are both factors predisposing to ulceration of the mucous membranes of the gastroesophageal tract and duodenum. In particular, the increase in acid and pepsin secretions increases the insults to the mucosa and predisposes it to a continuous pro-inflammatory state. Various types of ulcers can be identified, which are distinguished by their location in the digestive tract: duodenal ulcers are located in the duodenum, the first section of the small intestine; gastric ulcers are located within the gastric wall, and esophageal ulcers are located in the esophageal mucosa. The latter typically result in reflux. An ulcer is a necrotic lesion that penetrates the whole thickness of the mucosa and is characterised by severe epigastric pain. A peptic ulcer occurs due to an imbalance in the mechanisms that regulate tissue homeostasis. In particular, there is an increase in the aggressive factors, which break the mechanisms that the gastric mucosa uses to protect itself, and a decrease in the defensive factors, which normally contribute to maintaining the integrity of the mucosa. The increase in acid and pepsin secretions that increases the insults to the mucosa and predisposes it to a continuous pro-inflammatory state should be mentioned, in the first place, among the aggressive factors that contribute to damaging the mucosa. The presence of *Helicobacter pylori* contributes to damaging the mucosa as it disrupts the immune and inflammatory mechanisms implemented by cells in response to an insult. Other factors involved in the onset of peptic ulcers are: the intake of non-steroidal anti-inflammatory drugs which, by inhibiting cyclooxygenases (both COX1 and COX2), reduce the formation of prostaglandins, with a protective effect on the mucosa; oxidative stress and increase in free radicals which favour the occurrence of the above-mentioned imbalance between aggressive factors and protection mechanisms; inhibition of cell proliferation and infiltration of pro-inflammatory molecules. Pharmacological therapies commonly used to reduce hyperacidity, improve digestion and protect the gastric mucosa from any aggressive insults involve the use of an antisecretory agent, such as $H_2$ receptor antagonists and pump inhibitors, a mucosal protective agent, such as prostaglandin analogs, an antacid, or a complex bismuth salt. In the long run, therapies with the aforementioned agents exhibit several side effects. The use of cimetidine, an $H_2$ receptor antagonist, for example, resulted in the occurrence of gynecomastia. Toxicological studies on omeprazole revealed that the use of the above pump inhibitor may have, as a side effect, the formation of carcinoids, i.e., endocrine tumours of the stomach which are generated by an increase in the proliferation of enterochromaffin cells. As a result, in recent times, attention has grown towards safer alternatives that have fewer side effects, and natural products are advancing in the pharmaceutical industry as potential new sources of bioactive molecules. Many natural substances have already proven effective in gastroprotection, as well as safe and well-tolerated. Therefore, the object of the present invention is to provide a valid alternative to currently available therapies for gastroesophageal reflux, for protecting the gastric mucosa, promoting the digestive process, improving situations of abdominal discomfort and heartburn, as well as preventing the onset of a more serious pathological disorder.

Hyaluronic acid is a high molecular weight glycosaminoglycan consisting of repeating disaccharide units. Each disaccharide consists of one molecule of D-glucuronic acid and N-acetyl-glucosamine. Hyaluronic acid is the main component of the extracellular matrix in many tissues. Fibroblasts are the main components responsible for the secretion of hyaluronic acid in the extracellular matrix. This glycosaminoglycan is involved in many key processes within tissues and cells. These include cell signalling mechanisms, tissue regeneration and wound repair processes, tissue morphogenesis and differentiation processes, organization of the cellular matrix. Clinically, it is used in the treatment of various pathological conditions, in particular it is widely used to promote the healing of wounds, ulcers and aphthae. Thanks to its hydrophilic and hydrodynamic properties, hyaluronic acid is able to retain water, and therefore play an important structural role in cells. Its high molecular weight structure and its ability to form macro-aggregates, known as proteoglycans, give it an efficient anti-hyaluronidase action, which results in protection of the same proteoglycans in the connective tissue. Under normal conditions, proteoglycans contribute to making the extracellular matrix resistant to compressive forces and represent an effective barrier against bacterial infections. When a tissue is damaged, the use of high molecular weight hyaluronic acid helps rebuild the barrier, and the formation of proteoglycans contributes to the reduction of edema formation. In addition to the above-mentioned properties, there is an interesting regulatory action on the anti-inflammatory process. Hyaluronic acid acts by exerting a scavenger effect on prostaglandins and metalloproteinases, thereby reducing inflammation mediators, reactive oxygen species and free radicals, and reduces leukocyte infiltration phenomena, which are factors potentially involved in ulceration processes. Several pre-clinical and clinical studies praise the protective and healing properties of hyaluronic acid, highlighting its benefit in the prevention and treatment of peptic ulcers. The positive action of this glycosaminoglycan in the treatment of ulcers is ascribed to a direct barrier effect on the mucosa and to the activation of the mechanisms of repair and healing of the sub-mucosal connective tissue. Therefore, hyaluronic acid would be effective both in protecting the mucous membrane of the stomach and esophagus in all cases of hyperacidity and preventing the formation of damage and ulceration, and in promoting healing and reducing the uncomfortable sensations felt in the event that the integrity of the epithelium has already been affected by acid hyper-secretion. Furthermore, the anti-inflammatory action on the injured mucosa allows the pro-inflammatory mechanisms triggered in damaged tissues to be slowed down, edema formation to be reduced, healing to be accelerated, and burning-related symptoms to be immediately relieved.

A pre-clinical study on experimental animals tested the efficacy of two hyaluronic acid formulations in an ethanol-induced gastric ulcer model. The selected animals were randomized and divided into four groups: control group, positive control group (20mg/kg omeprazole), group A (240mg/100g high molecular weight hyaluronic acid), and group B (0.8% hyaluronic acid gel). The animals were fasted for 48 hours and were administered with absolute ethanol (5mL/kg) one hour after pretreatment with the above-mentioned substances, in order to induce ulceration. After approximately one hour, the animals were sacrificed and the stomachs collected and opened along the greater curvature. The area of the lesion in terms of $mm^2$ was assessed on the tissues examined. The results obtained from the test showed that pre-treatment with the gel significantly reduces the area of the gastric ulcers compared to the control. Pretreatment with high molecular weight hyaluronic acid had the most prominent effect on mucosal protection compared to both the control and omeprazole. Histological assessment showed that hyaluronic acid reduces gastric mucosa damage, edema and leukocyte infiltration.

A clinical study assessed the protective effect of hyaluronic acid in patients with gastric or duodenal ulcer by injecting a 0.2% hyaluronic acid solution into the sub-mucosal area surrounding the bleeding site. The injected solution was prepared by using sodium hyaluronate and brine. Patients included in the study received 20 mL of solution injected into the bleeding area, and in most of the cases considered, the bleeding was stopped already at the first administration and a persistent control of the bleeding was reported during the follow-up period.

Another clinical study assessed the effect of a hyaluronic acid-based formulation in combination with proton pump inhibitors in patients with gastroesophageal reflux. The 154 patients included in this multicenter, randomized, double-blind study were divided into two groups. The treated group (n = 76) received one stick per day of the test formulation (equivalent to 10 mL) for 14 days. The placebo group (n = 78) received a placebo for the same period of time. The study assessed the remission of symptoms (intended as retrosternal pain, heartburn, acid regurgitation and sour taste in the mouth) and the quality of life of the patients. Data from this study show that a greater number of patients achieved total remission of symptoms following combined treatment. In addition, the use of mucosal protective agents appears to prolong the remission period and delay relapse.

The kiwi fruit is an edible berry representing the fruit of plants belonging to the genus Actinidia. In particular, the two most known variants are *Actinidia chinensis Planch.* and *Actinidia deliciosa (A. Chev.) C.F.Liang & A.R. Ferguson.*

*Actinidia chinensis* is a woody perennial plant belonging to the Actinidiaceae family. It is native to China but today it is also cultivated in New Zealand, United States, Greece, Italy, Chile, France, Japan and Korea, and is mainly distributed in temperate or temperate-warm zones. The kiwi fruit has great economic, nutritional and medicinal significance in terms of production and use. Generally, the fruit is oval in shape, the skin is yellow to green and covered with a yellowish brown down. It can weigh approximately 120 g and can be eaten fresh, although on the market there are many kiwi fruit-based food preparations, juices, yoghurts, wines, dehydrated or candied fruits. The whole *Actinidia chinensis* plant has always played a leading role in traditional Chinese folk medicine. The fruit, with a sweet and sour taste, is claimed to have beneficial effects on the spleen, stomach and kidneys, and the ability to improve conditions such as dyspepsia, loss of appetite and vomiting. The branches and leaves were used to treat joint pain, bleeding and ulcers. The roots and the bark, with a bitter and astringent taste, were used to improve the micro-circulation and for their anti-inflammatory and draining effect, for example, applicable to rheumatoid arthritis. Today there is a lot of scientific evidence that confirms many of the properties that have always been attributed to this plant and that introduce the possibility of other promising applications. Recent studies carried out on Actinidia have suggested that this plant can be promising for its anti-tumour, antioxidant, anti-inflammatory, antimicrobial, immunoregulatory, hypolipidemic and anti-diabetic action, and for its protective action on the cardiovascular system and hypnotic action. The results obtained from complex chemical analyses showed the complex nutritional profile of the kiwi fruit, which is rich in phytocompounds such as polyphenols, triterpene compounds and derivatives thereof, carotenoids, polysaccharides, amino acids, vitamins, essential oils and micronutrients. Among all the compounds identified, the main bioactive molecules are phenolic compounds, triterpenes, vitamin C, vitamin E, fibres and microelements. In addition to the already mentioned compounds to which antioxidant and anti-inflammatory actions are mainly ascribed, which can be applied in different fields, it should be noted that the kiwi fruit is rich in proteases, of which the most abundant is actinidine. Proteases are enzymes that help cut proteins and are involved in digestive processes. The presence of proteases in the kiwi fruit apparently explains their beneficial effect on the gastroesophageal tract, since by promoting the digestive processes they reduce the gastric emptying time. The slowing down of the gastric emptying, in fact, causes an increase in the time of contact of the mucosa with the acidic digestive gastric juices and increases the risk of gastroesophageal reflux. An *in vitro* study measured the effect of the kiwi fruit on protein hydrolysis by mimicking the acidic and duodenal digestion. In this experiment, 600 mg of a protein sample were subjected, with or without kiwi fruit treatment, to a simulated gastric digestion for 60 minutes at 37°C with various pepsin and hydrochloric acid concentrations using a pH range between 1.3 and 6.2. For the duodenal digestion, the samples were incubated for 120 minutes at pH 6.4. The experiment showed that protein digestion mainly occurs at the gastric level and that the use of kiwi fruit enzymes doubles protein hydrolysis in the gastric environment, measured as a percentage of ammonia nitrogen in the supernatant.

A preclinical study in rats assessed the effect of several kiwi fruit variants on gastric emptying and protein digestion. Kiwi fruit variants used for the test were *Actinidia chinensis var. deliciosa,* with a high content of actinidine, and *Actinidia chinensis var. chinensis,* with a low content of actinidine. The action of these variants was tested on different protein samples from different sources to which two different kiwi fruit extracts were added. 24 rats were selected for assessing gastric emptying and 8 rats were used for each treatment. A total of 14 protein diets were carried out: 2 controls, 6 with *A. deliciosa* added and 6 with *A. chinensis* added. For assessing gastric emptying, more than one diet is administered to each group of 8 rats. On the day of the test, rats were administered with 2 mL of one of the above mentioned mixtures, and the transit was recorded by magnetic resonance spectroscopy for 150 minutes. The results obtained show that actinidine accelerates gastric emptying to a greater extent for some protein sources than for others, particularly for beef and zein proteins. To assess the effect on protein digestion, 104 rats were selected and divided into groups and subjected to treatments as indicated above. At the end of the supplementation, the rats were sacrificed and chyme samples were taken and examined for their protein digestion levels in terms of free and total amino groups. The results obtained confirmed the previous data showing that actinidine increases gastric protein digestion.

A randomized, cross-over, pilot clinical trial was conducted to assess the effect of taking two kiwi fruit varieties on gastric emptying in terms of satiety and overall gastric comfort. Ten healthy subjects between the ages of 21 and 48 were selected for the following trial. The trial was set up as follows: the selected subjects were asked to show up on the day of the test after fasting from food and liquids from the previous evening. The test was carried out by administering to each a high protein content meal and two kiwi fruits of two different varieties (var. chinensis and var. deliciosa). Before and after meals, and at hourly intervals for the following 5 hours, subjects were asked to complete a questionnaire on their sense of satiety, bloating and general well-being. To assess the gastric emptying time, subjects were administered a Smartpill, i.e., a wireless cylindrical capsule that measures the pH and temperature values in real time and transmits the recorded data to a receiver attached to the clothes of each subject. Based on the drastic change in pH, the device allows the recording of the passage from the gastric to the intestinal environment. The same clinical procedure was repeated three times; each test was separated from the previous one by a period of at least 7 days. The results obtained have shown that both the kiwi fruit variants have an almost superimposable effect as regards the gastric emptying time.

Ginger (*Zingiber officinale* Roscoe) is a biennial or perennial herbaceous plant belonging to the Zingiberaceae family, native to South Asia, consisting of 49 genera and 1300 species. The stems of the plant can be up to 1 metre high and have lanceolate leaves approximately 20 centimetres in length. The term "ginger" commonly refers to the rhizome of the plant, which is commonly used as a cooking spice in Asian countries, or as a fragrance in the food industry. Chemical analysis results indicate that ginger contains more than 400 different compounds. The main constituents in the rhizome are carbohydrates (50-70%), lipids (3-8%), terpenes and phenolic compounds. Terpene compounds in ginger include zingiberene, β-bisabolene, α-farnesene and α-curcumene, whereas the phenolic compounds include gingerols, paradols and shogaols. Gingerols and shogaols are the main components and are present in greater quantities than others. Amino acids, fibres, proteins, phytosterols, vitamins (nicotinic acid and vitamin A) and minerals are also present in the rhizome. Ginger and its components have varied pharmacological activities affecting various types of disorders: gastrointestinal, antioxidant, cardiovascular, analgesic and anti-inflammatory activities. In particular, the latter properties (analgesic and anti-inflammatory) are found in studies carried out in mice with the "hot-plate" and "acetic acid" methods as regards the analgesic activity, and with the "induced pedal edema" method as regards the anti-inflammatory activity. The results from these studies have shown that, in a comparison between ginger and commonly used drugs (such as morphine, diclofenac, chlorpramide), ginger shows anti-inflammatory and analgesic activities, without however causing the side effects that instead characterize the above mentioned drugs.

Further scientific evidence places ginger at the centre of research aimed at intestinal well-being, in fact, it has proved useful in reducing the pressure on the esophagus, reducing intestinal cramps, preventing dyspepsia and flatulence. In addition, it acts on the gastroesophageal transit speed, by relaxing the muscles of the affected tract. This already broad therapeutic spectrum is enriched by a proven anti-emetic action, which is useful because a more or less acute sense of nausea and vomiting is often associated with the gastrointestinal disorders.

The anti-ulcer activity of ginger was assessed in an *ex-vivo* study in albino rats. Two categories of cytodestructive agents favouring the ulcerative state were used in this study: the first agent consists of an 80% ethanol, 0.6M HCl, 0.2M NaOH, 25% NaCl solution, the second agent consists of a drug selected from indomethacin, aspirin or reserpine. The modus operandi of these tests consisted in pretreatment with ginger at a concentration of 500 mg/kg body weight, subsequent insult with a cytotoxic agent 30 minutes later, and sacrifice of the guinea pigs accompanied by analysis of the tissue damage to the stomach. In any case, the results showed a clear cytoprotective effect of ginger with a consequent anti-ulcer action. The anti-ulcer and cytoprotective actions are added with that relating to an increased gastric emptying rate. In this regard, an *in vivo* study was carried out on 24 healthy volunteers, who fasted for 8 hours and were then divided into two groups. After the 8-hour fasting, the first group was given 3 placebo capsules, whereas the second group was given 3 ginger-based capsules (for a total content of 1200 mg). After this administration, all volunteers were fed a low-nutrient meal, after which gastric activity was monitored by ultrasound. The results showed that the gastric emptying rate in the volunteers of the ginger group was significantly higher than the emptying rate of the placebo group, which led to evidence that ginger contributes to gastrointestinal motility.

The real added value of ginger is its anti-emetic action, investigated in a study carried out on 120 women in post-operative conditions. The study compared 3 groups: placebo group, ginger group, and metoclopramide group. What emerged was the real efficacy of ginger and metoclopramide compared to the untreated group (placebo), and that the two substances are superimposable in terms of anti-emetic efficacy. In this case, too, ginger is considered as the preferred substance compared to metoclopramide due to the lack of side effects.

EP 2545925 discloses compositions comprising hyaluronic acid for the treatment of gastro-intestinal disorders.

WO 2010/083968 and WO 2010/083967 disclose compositions comprising Zingiber officinale for the treatment of gastro-intestinal disorders.

CN 107149658 discloses compositions comprising Actinidia chinensis for the treatment of gastro-intestinal disorders.

WO 2017/158041 discloses the combination of hyaluronic acid and a lipophilic extract of ginger (i.e., *Zingiber officinale),* the therapeutic indication mentioned therein is the treatment of disorders of the oral cavity, upper respiratory tract and oesophagus.

One object of the present invention is therefore a composition as defined in appended claim 1. Further features and advantages of the invention are defined in the dependent claims. All claims form an integral part of the present invention.

In a preferred embodiment, the *Actinidia chinensis* extract is present in the composition in an amount ranging from 0.01 to 50% by weight, preferably from 0.05 to 30% by weight, even more preferably from 0.1 to 10% by weight based on the total weight or volume of the composition.

In another preferred embodiment, the *Zingiber officinale* Rosc. extract is present in an amount ranging from 0.01 to 50% by weight, preferably from 0.05 to 30% by weight, even more preferably from 0.1 to 10% by weight based on the total weight or volume of the composition.

Within the scope of the present invention, the term "extract" refers to a preparation obtained from any part of the plant, including the maceration and/or processing thereof.

In a further preferred embodiment, hyaluronic acid or a salt thereof is present in an amount ranging from 0.01 to 50% by weight, preferably from 0.05 to 30% by weight, even more preferably from 0.1 to 10% by weight based on the total weight or volume of the composition.

In yet another preferred embodiment, the composition of the invention is a pharmaceutical composition, a food supplement, a medical device, a simple feed, a complementary feed, a complete feed, formulated into an oral dosage form, which is preferably solid, semi-solid or liquid. An oral pharmaceutical form is preferred.

Pharmaceutical forms for oral administration include, but are not limited to, a stick gel, a tablet, a capsule, a powder, a granulated orosoluble powder, a granulated orosoluble syrup, a solution, a suspension and a pellet.

The above concentrations of the active ingredients refer to the quantity of each of the indicated active ingredients in the final pharmaceutical embodiment, including all the excipients, and, depending on whether the pharmaceutical form is liquid, solid or semi-solid, are expressed as w/w or w/v. For example, in the case of the most preferred pharmaceutical form, i.e., the stick gel for oral administration, concentrations are expressed as w/v.

Suitable pharmaceutically acceptable carriers and/or excipients are those commonly known to those skilled in the art for the preparation of compositions for oral administration such as powders, granulates, capsules, tablets, solutions, suspensions, etc. By way of non-limiting example, such pharmaceutically acceptable carriers and/or excipients are selected from the group consisting of diluents (e.g., dibasic calcium phosphate, lactose, microcrystalline cellulose and cellulose derivatives), thickeners (e.g., gums, hydroxypropylmethylcellulose and other cellulose derivatives), sweeteners (e.g., sorbitols, mannitol and other polyols, acesulfame K, aspartame, cyclamates, saccharin, sucralose), lubricants (e.g., magnesium stearate, stearic acid, waxes), dispersants, surfactants (e.g., sodium lauryl sulphate and polysorbates), flavourings, adsorbents (e.g., silica gel, talc, starch, bentonite, kaolin), glidants and anti-adherents (e.g., talc, colloidal silica, maize starch, silicon dioxide), dyes (e.g., iron oxides), opacifiers (e.g., titanium oxide), antioxidants, binders (e.g., gums, starch, gelatin, cellulose derivatives, sucrose, sodium alginate), disintegrants (starch, microcrystalline cellulose, alginic acid, crospovidone), plasticizers (e.g., ethyl cellulose and other cellulose derivatives, acrylates and methacrylates, glycerol and sorbitol), preservatives (e.g., parabens, sulfur dioxide), viscosifiers, emulsifiers, humectants, wetting agents, chelating agents and/or any combination thereof.

A further aspect of the invention is the above described composition for use in the therapeutic treatment of gastrointestinal disorders in a subject (a human or non-human animal), wherein the gastrointestinal disorders are selected from the group consisting of gastritis, gastroesophageal reflux, gastric hyperacidity, delay in the process of gastric emptying, heartburn and dyspepsia.

In a preferred embodiment, treatment comprises administering hyaluronic acid or a salt thereof in a therapeutically effective amount comprised between 0.1 and 3000 mg, preferably between 1 mg and 2000 mg/die, more preferably between 10 mg and 1000 mg/die.

In another preferred embodiment, treatment comprises administering the *Actinidia chinensis* extract in a therapeutically effective amount comprised between 0.1 and 5000 mg/die, preferably between 5 mg and 3500 mg/die, more preferably between 10 mg and 1000 mg/die.

In a still further preferred embodiment, treatment comprises administering the *Zingiber officinale* Rosc. extract in a therapeutically effective amount comprised between 0.1 mg and 5000 mg/die, preferably between 5 mg and 3500 mg/die, more preferably between 10 mg and 1000 mg/die.

All of the above embodiments can be combined with each other.

As indicated above, the composition of the present invention is effective in the protection of the gastrointestinal mucosa and in the prevention and treatment of diseases from gastric disorders in humans or other animals (for example dogs or cats). the gastric disorders suitable to be treated with the composition of the invention are gastritis, gastroesophageal reflux, gastric hyperacidity, delay in the gastric emptying process, heartburn, and dyspepsia. The combination of active ingredients that characterises the composition of the present invention allows:

- Gastroprotection
- Healing of gastric lesions and ulcers
- Reduction of tissue inflammation and oxidation phenomena
- Reduction of the gastric emptying time
- Neutralization of gastric hyperacidity
- Resolution of symptoms related to gastroesophageal reflux
- Anti-emetic action

The composition of the present invention is therefore a valid aid in protecting the gastric mucosa from aggressive agents which may increase the risk of tissue ulceration, and contributes to the repair of damaged tissue. The protective action of the composition of the invention is due to the synergistic action of the components that make it up. The *Actinidia chinensis Planch.* extract, besides promoting digestion and reducing gastric emptying time, performs an antioxidant and anti-inflammatory action. Hyaluronic acid exerts a barrier effect on the mucous membranes and contributes to the activation of the healing mechanisms in the submucosal connective tissue. With its anti-inflammatory action, it also contributes to reducing edema formation and relieving symptoms. Ginger has a cytoprotective action and acts on gastrointestinal motility, by speeding up gastric emptying; this is accompanied by anti-inflammatory and antioxidant characteristics, while the anti-emetic effect brings added value, as it acts on one of the symptoms characterizing the complex symptoms linked to disorders of the gastrointestinal system.

The action of the individual components of the composition of the invention and that of the combination thereof can be assessed by *in vitro* and/or *in vivo* tests. The latter parameter will be verified if at least one of the therapeutic effects described above satisfies the principle that the sum of the activities expressed by the individual components is less than the activity expressed by the components in combination.

The antioxidant activity of the composition of the invention, comprising the three combined active ingredients, and that of the three active ingredients taken individually, can be determined by radical scavenging activity evaluation methods. An example of a test involves the use of DPPH (2,2-diphenyl-1-picrylhydrazyl) as a reagent. Ascorbic acid is prepared and used as a standard sample. Different concentrations of the composition of the invention are tested, to which a DPPH solution in ethanol is added, and the mixture is incubated at room temperature in the dark. The scavenging activity is determined by measuring the absorbance at a wavelength of 517 nm. The percentage of scavenging activity is calculated by using the following formula:

$$\text{Radical scavenging activity } (\%) = [1-(A_{\text{sample}} / A_{\text{blank}})] \times 100.$$

*In vitro* tests on cell cultures are useful for assessing the protective effect of the composition of the invention. For example, GES-1 human gastric epithelial cells cultured in complete DMEM growth medium are used and incubated at 37°C and in a 5% $CO_2$ humid atmosphere. After a 24-hour treatment with different concentrations of the test substances, an MTT assay is carried out to assess the cell viability and the cytotoxicity of the active ingredients of interest. Absorbance is measured at 570 nm following a short period of incubation with the reagent. To assess the antioxidant effect of the above formulation, intracellular ROS levels are assessed by DCFH-DA (2,7-dichlorofluorescein diacetate) assay, the differently treated cells are washed and incubated with DCFH-DA and the fluorescence intensity is measured. The total antioxidant capacity (T-AOC) is then assessed by the ABTS method using a suitable kit and measuring the optical density of the supernatant at 420 nm. In order to analyse the levels of SOD, GSH-Px, MDA and LDH, cells treated with the test substances at different concentrations are sonicated. The supernatant is collected and used to determine the levels of SOD, GSH-Px, MDA and LDH with appropriate kits.

An *in vitro* inflammation model can be used to assess the anti-inflammatory action of the composition of the invention. For example, RAW264.7 murine macrophages stimulated with bacterial LPS are used. Cells are cultured in complete DMEM growth medium and incubated at 37°C in a 5% $CO_2$ humid atmosphere. 1 hour after treatment with different concentrations of the test formulation, LPS is added for a total time of 24 hours. At the end of this time, the cell viability and the cytotoxicity of the active ingredients of interest are assessed by the MTT assay. NO content in the growth medium is also determined by using the Griess reagent. NO concentration is assessed by measuring the absorbance at 540 nm and the calibration curve is obtained by dilution with $NaNO_2$. Each treatment is performed in triplicate. In order to determine the anti-inflammatory effect, the cells are treated with different concentrations of the test formulation for 1 hour, after which LPS is added and the cells are incubated again for 1 hour and for 18 hours. At the end of the treatment, the cells are collected and protein extraction is carried out. The samples thus obtained are assayed by western blotting so as to determine the levels of inflammatory markers such as iNOS, COX-2, TNF-$\alpha$, IL-1$\beta$, p-NF-kB p65, p-IkB$\alpha$.

An *in vitro* test for the inhibition of the $H^+/K^+$ ATPase enzyme, i.e., the gastric proton pump, can be carried out in order to verify the antacid activity of the composition of the invention comprising a kiwi fruit extract, a ginger extract and hyaluronic acid. In order to obtain the enzymatic samples to be tested, gastric parietal cells can be obtained from sheep stomach, which are then homogenized and centrifuged for 10 minutes. The supernatant (enzyme extract) is then collected in order to determine the inhibitory activity of the test substances. The reaction mixture containing the enzyme extract and the formulation containing the three active ingredients of interest is preincubated for 60 minutes at 37°C. The reaction is induced by adding ATP (substrate), $MgCl_2$ and KCl. After 30 min of incubation at 37°C, the reaction is stopped by adding a mixture containing 4.5% ammonium molybdate and 60% perchloric acid, then it is centrifuged for 10 minutes and the inorganic phosphate produced by the reaction is measured spectrophotometrically at the wavelength of 660 nm following the Fiske-Subbarow method. mQ water, ammonium molybdate and ANSA are added to the supernatant and the mixture is left to stand for 10 minutes at room temperature. The absorbance of the released inorganic phosphate is measured at 660 nm. The enzymatic activity is calculated as micromoles of Pi (inorganic phosphate) released per hour at the various doses of the mixture of the two extracts. The percentage of enzymatic inhibition is calculated by using the following formula:

$$\text{Percentage of inhibition} = [\text{Activity}_{\text{(control)}} - \text{Activity}_{\text{(test)}}/\text{Activity}_{\text{(control)}}] \times 100.$$

The effectiveness of the composition of the present invention can also be assessed by an *in vivo* test on experimental animals in accordance with the directives of the European Community and the Ministry of Health and approved by an Ethics Committee. A good example of experiments of this type are those carried out in Sprague-Dawley mice pretreated with the test substances. Following pretreatment, a high dose of an ulcerative agent is administered, and after a variable incubation period of 1 to 5 hours, the animals are sacrificed and the stomach of each is removed and opened along the greater curvature. The gastric mucosa is assessed for the number and size of the ulceration areas (expressed in $mm^2$). Assessment of the ulceration areas is carried out as described above. The gastric secretions are assessed by collecting the volume of the gastric juice, which is used for measuring the pH, pepsin activity (by means of a special kit) and total acidity by titration with sodium hydroxide. The amount of mucus produced can be assessed by scraping the mucosa, and the collected mucus is weighed using a precision electronic balance. *Ex-vivo* tests are performed on the collected tissues. Isolated tissue sections are homogenized and the obtained samples are used for quantizing the MDA, SOD, CAT, and GPx levels. MDA levels are measured by testing the lipid peroxidation levels. For this purpose, a reactivity test is carried out by using TBA (thiobarbituric acid) and measuring the absorbance at 532 nm with a UV-visible spectrophotometer. SOD levels are measured by epinephrine assay and by assessing the absorbance at 480 nm at the end of the assay. CAT activity is assessed by means of a special kit. Free iron levels are assessed in blood samples taken from the tested animals at the time of the sacrifice by colorimetric measurement using ferrozine. A variant of the above assay involves the use of EDTA as the reaction standard. Plasma calcium levels are measured by a colorimetric method using the reaction with cresolphthalein and measuring the absorbance of the complex formed at 570 nm. Plasma samples are also assessed for their anti-inflammatory action by determining the levels of TNF-$\alpha$, IL,-1$\beta$, gastrin, NO and PGE2.

A further *in vivo* test on animals, which is useful for assessing the gastro-protective activity of the composition of the present invention, comprises the following protocol. The selected mice are fasted for 18 hours prior to the operation to induce an esophagitis condition. Two hours before the operation the mice are treated with the test substances. The mice are anesthetized and a 2 cm incision is made in the central part of the abdomen in order to expose the stomach, after which the pylorus is ligated to induce reflux, while maintaining the vagal nerve intact. After 5 hours of operation, all mice are sacrificed and the esophagus is immediately removed, washed with saline and photographed to evaluate mucosal lesions. At this point, the anti-inflammatory activity of the substances is assessed by determining the levels of inflammatory markers such as COX-2, IL-1$\beta$, TNF$\alpha$, and IkB$\alpha$ and NF-kB, by western blotting. Furthermore, in order to determine the histamine content, a blood sample is taken from the supraorbital plexus using the microcapillary technique and the plasma is separated. The plasma is treated with perchloric acid and centrifuged for 30 minutes at 4°C, and the supernatant is used to assess the histamine content by HPLC (expressed in IU/mg protein).

Further, the effect of the composition of the invention on the gastric emptying time can be assessed, for example, by following the model described by Smits and Lefebvre (1996). Male mice are fasted for 18 hours and then fed 20 minutes prior to the start of the experiment in order to ensure that the food content in the stomach is as similar as possible. The composition of the invention comprising *Actinidia chinensis* extract, *Zingiber officinale* Rosc. extract and hyaluronic acid and/or a salt thereof is administered orally; after 30 minutes, a marker (a suspension containing 50 mg of phenol red in 100 ml of 1.5% carboxymethylcellulose) is administered to the animals to assess the gastric emptying rate. After 20 minutes, the animals are sacrificed and the stomach is removed. The stomach then placed in tubes containing saline, after 20 seconds of stirring, is treated with 1M NaOH in each tube so as to obtain the maximum colorimetric intensity. At this point, a spectrophotometric analysis is carried out at a wavelength of 560 nm. The percentage of gastric emptying is calculated by using the following formula:

Gastric emptying (%) = [1-(amount of phenol red present in the stomach after 20 min)/(amount of phenol red present in the stomach at time 0)] x 100.

Assessment of the gastric emptying can also be performed by Magnetic Resonance Spectroscopy (MRS). This is a non-invasive technique that allows more than one experiment to be performed on the same animal. The mice must weigh between 275 and 315 g. For this experiment, the mice are given a standard diet with the addition of an indigestible marker (titanium dioxide) and the treatment assigned for that study group. Prior to MRS analysis, the mice are fasted for 14 hours without food or water, then 1 g of the assigned diet is given to each group. Rats are then scanned every 25 min/150 min.

Other parameters to be analysed for the evaluation of the efficacy of the composition of the invention are the reflux and gastric secretion extents. These evaluations can be carried out after pyloric ligation. Before the start of the experiment, the animals are fasted for 24 hours, while free access to water is granted. The animals are then anesthetized and subjected to pyloric ligation surgery. A longitudinal myotonia 1 centimetre thick is made at the gastroesophageal junction in order to promote gastroesophageal reflux. The treatment is administered immediately after the pyloric ligation surgery. At this point, the mice are fasted for 24 hours and then sacrificed in a $CO_2$ saturated atmosphere, followed by removal of the esophagus. The esophageal mucosa is surgically separated from the muscle layer in order to assess any lesions. The area of the esophageal mucosa exhibiting lesions is assessed by microscopic analysis. Other examinations may concern the stomach and its contents, the pH, any lesions in the gastric mucosa. In order to examine the gastric juice, it can be added with 2 ml of distilled water and centrifuged at 5000 rpm for 15 min at 4°C. After centrifugation, the supernatant is used for assessing the volume (mL/mouse), pH and acidity (mEq/L). Total acidity is assessed by titration with 0.01 N NaOH at pH=7 (using phenolphthalein as the indicator). Total acidity is thus expressed as mequiv. [H$^+$]/ml/4 h. The evaluation of the gastric lesions is carried out by opening the stomach by cutting along the greater curvature and stretching it out on a polystyrene support. The total area of the gastric lesions is measured, and the degree of severity of the gastric lesions is expressed by means of a gastric lesion index (mm$^2$).

**EXAMPLES**

Some examples of quantities per single dosage unit of the active components of the compositions object of the present invention are given for illustrative and non-limiting purposes.

EXAMPLE 1: 10 mL stick gel for oral administration

| Active ingredient | Quantity per single dosage unit |
|---|---|
| Actinidia chinensis | 30 mg |
| Zingiber officinale Rose. | 20 mg |
| Sodium hyaluronate | 33 mg |

EXAMPLE 2: 10 mL stick gel for oral administration

| Active ingredient | Quantity per single dosage unit |
|---|---|
| Actinidia chinensis | 10 mg |
| Zingiber officinale Rose. | 30 mg |
| Hyaluronic acid | 25 mg |

EXAMPLE 3: Orosoluble powder

| Active ingredient | Quantity per single dosage unit |
|---|---|
| Actinidia chinensis | 50 mg |
| Zingiber officinale Rose. | 60 mg |
| Hyaluronic acid | 100 mg |

EXAMPLE 4: Granules for oral use

| Active ingredient | Quantity per single dosage unit |
|---|---|
| Actinidia chinensis | 25 |
| Zingiber officinale Rose. | 15 |
| Hyaluronic acid | 50 |

## Claims

1. A composition comprising hyaluronic acid or a salt thereof, *Actinidia chinensis* extract and *Zingiber officinale* extract, optionally in admixture with one or more pharmaceutically acceptable carriers and/or excipients.

2. The composition according to claim 1, wherein said pharmaceutically acceptable carriers and/or excipients are selected from the group consisting of diluents, thickeners, sweeteners, lubricants, dispersants, surfactants, flavourings, adsorbents, glidants, antiadherents, dyes, opacifiers, antioxidants, binders, disintegrants, plasticizers, preservatives, viscosifiers, emulsifiers, humectants, wetting agents, chelating agents and any combination thereof.

3. The composition according to claim 1 or 2, comprising from 0.01 to 50% by weight of hyaluronic acid, preferably from 0.05 to 30% by weight of hyaluronic acid, even more preferably from 0.1 to 10% by weight of hyaluronic acid.

4. The composition according to any of claims 1 to 3, comprising from 0.01 to 50% by weight of *Actinidia chinensis* extract, preferably from 0.05 to 30% by weight of *Actinidia chinensis* extract, even more preferably from 0.1 to 10% by weight of *Actinidia chinensis* extract.

5. The composition according to any of claims 1 to 4, comprising from 0.01 to 50% by weight of *Zingiber officinale* extract, preferably from 0.05 to 30% by weight of *Zingiber officinale* extract, even more preferably from 0.1 to 10% by weight of *Zingiber officinale* extract.

6. The composition according to any of claims 1 to 5, which is selected from the group consisting of a pharmaceutical composition, a food supplement, a medical device, a simple feed, a complementary feed, a complete feed, in an oral dosage form, which is preferably solid, semi-solid or liquid.

7. The composition according to any of claims 1 to 6, in a dosage form selected from the group consisting of a stick gel, a tablet, a capsule, a powder, a granulated orosoluble powder, a granulated orosoluble syrup, a solution, a suspension and a pellet.

8. The composition according to any of claims 1 to 7, for use in the therapeutic treatment of gastrointestinal disorders in a subject, wherein the gastrointestinal disorders are selected from the group consisting of gastritis, gastroesophageal reflux, gastric hyperacidity, delay in the process of gastric emptying, heartburn and dyspepsia.

9. The composition for use according to claims 8 , wherein the subject is a human being or another animal.

10. The composition for use according to claim 8 or 9, wherein the treatment comprises administering a therapeutically effective amount of hyaluronic acid or a salt thereof comprised between 0.1 and 3000 mg/die, preferably comprised between 1 mg and 2000 mg/die, more preferably comprised between 10 mg and 1000 mg/die.

11. The composition for use according to any of claims 8 to 10, wherein the treatment comprises administering a therapeutically effective amount of *Actinidia chinensis* extract comprised between 0.1 and 5000 mg/die, preferably comprised between 5 mg and 3500 mg/die, more preferably comprised between 10 mg and 1000 mg/die.

12. The composition for use according to any of claims 8 to 11, wherein the treatment comprises administering a therapeutically effective amount of *Zingiber officinale* extract comprised between 0.1 mg and 5000 mg/die, preferably comprised between 5 mg and 3500 mg/die, more preferably comprised between 10 mg and 1000 mg/die.

## Patentansprüche

1. Zusammensetzung, umfassend Hyaluronsäure oder ein Salz davon, Extrakt von *Actinidia chinensis* und Extrakt von *Zingiber officinale,* optional in Beimischung mit einem oder mehreren pharmazeutisch verträglichen Trägern und/oder Hilfsstoffen.

2. Zusammensetzung nach Anspruch 1, wobei die pharmazeutisch verträglichen Träger und/oder Hilfsstoffe aus der Gruppe ausgewählt sind, bestehend aus Verdünnungsmitteln, Verdickungsmitteln, Süßungsmitteln, Schmiermitteln, Dispergiermitteln, Tensiden, Geschmacksstoffen, Adsorptionsmitteln, Gleitmitteln, Antihaftmitteln, Farbstoffen, Trübungsmitteln, Antioxidantien, Bindemitteln, Zersetzungsmitteln, Weichmachern, Konservierungsmitteln, Viskositätsmitteln, Emulgatoren, Feuchthaltemitteln, Netzmitteln, Chelatbildnern und einer beliebigen Kombination davon.

3. Zusammensetzung nach Anspruch 1 oder 2, umfassend zu 0,01 bis 50 Gew.-% Hyaluronsäure, vorzugsweise zu 0,05 bis 30 Gew.-% Hyaluronsäure, noch mehr bevorzugt zu 0,1 bis 10 Gew.-% Hyaluronsäure.

4. Zusammensetzung nach einem der Ansprüche 1 bis 3, umfassend zu 0,01 bis 50 Gew.-% Extrakt von *Actinidia chinensis,* vorzugsweise zu 0,05 bis 30 Gew.-% Extrakt von *Actinidia chinensis,* noch mehr bevorzugt zu 0,1 bis 10 Gew.-% Extrakt von *Actinidia chinensis.*

5. Zusammensetzung nach einem der Ansprüche 1 bis 4, umfassend zu 0,01 bis 50 Gew.-% Extrakt von *Zingiber officinale,* vorzugsweise zu 0,05 bis 30 Gew.-% Extrakt von *Zingiberofficinale,* noch mehr bevorzugt zu 0,1 bis 10 Gew.-% Extrakt von *Zingiber officinale.*

6. Zusammensetzung nach einem der Ansprüche 1 bis 5, die aus der Gruppe ausgewählt ist, bestehend aus einer pharmazeutischen Zusammensetzung, einem Nahrungsergänzungsmittel, einem Medizinprodukt, einem einfachen Futtermittel, einem Ergänzungsfuttermittel, einem Alleinfuttermittel, in einer oralen Dosierungsform, die vorzugsweise fest, halbfest oder flüssig ist.

7. Zusammensetzung nach einem der Ansprüche 1 bis 6 in einer Dosierungsform, die aus der Gruppe ausgewählt ist, bestehend aus einem Stiftgel, einer Tablette, einer Kapsel, einem Pulver, einem granulierten orolöslichen Pulver, einem granulierten orolöslichen Sirup, einer Lösung, einer Suspension und einem Pellet.

8. Zusammensetzung nach einem der Ansprüche 1 bis 7 zur Verwendung bei einer therapeutischen Behandlung von gastrointestinalen Störungen bei einem Subjekt, wobei die gastrointestinalen Störungen aus der Gruppe ausgewählt sind, bestehend aus Gastritis, gastroösophagealer Refluxkrankheit, Magenübersäuerung, Verzögerung der Magenentleerung, Sodbrennen und Dyspepsie.

9. Zusammensetzung zur Verwendung nach Anspruch 8, wobei das Subjekt ein Mensch oder ein anderes Tier ist.

10. Zusammensetzung zur Verwendung nach Anspruch 8 oder 9, wobei die Behandlung ein Verabreichen einer therapeutisch wirksamen Menge von Hyaluronsäure oder einem Salz davon umfasst, die zwischen 0,1 und 3000 mg/die, vorzugsweise zwischen 1 mg und 2000 mg/die, mehr bevorzugt zwischen 10 mg und 1000 mg/die liegt.

11. Zusammensetzung zur Verwendung nach einem der Ansprüche 8 bis 10, wobei die Behandlung das Verabreichen einer therapeutisch wirksamen Menge von Extrakt von *Actinidia chinensis* umfasst, die zwischen 0,1 und 5000 mg/die, vorzugsweise zwischen 5 mg und 3500 mg/die, mehr bevorzugt zwischen 10 mg und 1000 mg/die liegt.

12. Zusammensetzung zur Verwendung nach einem der Ansprüche 8 bis 11, wobei die Behandlung das Verabreichen einer therapeutisch wirksamen Menge von Extrakt von *Zingiber officinale* umfasst, die zwischen 0,1 mg und 5000 mg/die, vorzugsweise zwischen 5 mg und 3500 mg/die, mehr bevorzugt zwischen 10 mg und 1000 mg/die liegt.

**Revendications**

1. Composition comprenant de l'acide hyaluronique ou un de ses sels, de l'extrait *d'Actinidia chinensis* et de l'extrait de *Zingiber officinale,* éventuellement en mélange avec un ou plusieurs supports et/ou excipients pharmaceutiquement acceptables.

2. Composition selon la revendication 1, dans laquelle lesdits supports et/ou excipients pharmaceutiquement acceptables sont choisis dans le groupe constitué par diluants, épaississants, édulcorants, lubrifiants, dispersants, surfactants, arômes, adsorbants, glissants, antiadhésifs, colorants, opacifiants, antioxydants, liants, désintégrants, plastifiants, conservateurs, viscosifiants, émulsifiants, humectants, agents mouillants, agents chélatants et leurs combinaisons.

3. Composition selon la revendication 1 ou 2, comprenant de 0,01 à 50 % en poids d'acide hyaluronique, de préférence de 0,05 à 30 % en poids d'acide hyaluronique, encore plus préférablement de 0,1 à 10 % en poids d'acide hyaluronique.

**4.** Composition selon l'une quelconque des revendications 1 à 3, comprenant de 0,01 à 50 % en poids d'extrait *d'Actinidia chinensis,* de préférence de 0,05 à 30 % en poids d'extrait *d'Actinidia chinensis,* encore plus préférablement de 0,1 à 10 % en poids d'extrait *d'Actinidia chinensis.*

**5.** Composition selon l'une quelconque des revendications 1 à 4, comprenant de 0,01 à 50 % en poids d'extrait de *Zingiber officinale,* de préférence de 0,05 à 30 % en poids d'extrait de *Zingiber officinale,* encore plus préférablement de 0,1 à 10 % en poids d'extrait de *Zingiber officinale.*

**6.** Composition selon l'une quelconque des revendications 1 à 5, qui est choisie dans le groupe constitué d'une composition pharmaceutique, d'un complément alimentaire, d'un dispositif médical, d'un aliment simple, d'un aliment complémentaire, d'un aliment complet, sous une forme de dosage oral, qui est de préférence solide, semi-solide ou liquide.

**7.** Composition selon l'une quelconque des revendications 1 à 6, sous une forme de dosage choisie dans le groupe constitué d'un gel en bâtonnet, d'un comprimé, d'une capsule, d'une poudre, d'une poudre granulée orosoluble, d'un sirop granulé orosoluble, d'une solution, d'une suspension et d'une pastille.

**8.** Composition selon l'une quelconque des revendications 1 à 7, pour une utilisation dans le traitement thérapeutique de troubles gastro-intestinaux chez un sujet, dans lequel les troubles gastro-intestinaux étant choisis dans le groupe constitué par gastrite, reflux gastro-oesophagien, hyperacidité gastrique, retard dans le processus de vidange gastrique, brûlures d'estomac et dyspepsie.

**9.** Composition pour une utilisation selon la revendication 8, dans laquelle le sujet est un être humain ou un autre animal.

**10.** Composition pour une utilisation selon la revendication 8 ou 9, dans laquelle le traitement comprend l'administration d'une quantité thérapeutiquement efficace d'acide hyaluronique ou d'un de ses sels comprise entre 0,1 et 3000 mg/jour, de préférence comprise entre 1 mg et 2000 mg/jour, plus préférablement comprise entre 10 mg et 1000 mg/jour.

**11.** Composition pour une utilisation selon l'une quelconque des revendications 8 à 10, dans laquelle le traitement comprend l'administration d'une quantité thérapeutiquement efficace d'extrait *d'Actinidia chinensis* comprise entre 0,1 et 5000 mg/jour, de préférence comprise entre 5 mg et 3500 mg/jour, plus préférablement comprise entre 10 mg et 1000 mg/jour.

**12.** Composition pour une utilisation selon l'une quelconque des revendications 8 à 11, dans laquelle le traitement comprend l'administration d'une quantité thérapeutiquement efficace d'extrait de *Zingiber officinale* comprise entre 0,1 mg et 5000 mg/jour, de préférence comprise entre 5 mg et 3500 mg/jour, plus préférablement comprise entre 10 mg et 1000 mg/jour.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2545925 A
- WO 2010083968 A
- WO 2010083967 A
- CN 107149658
- WO 2017158041 A